Europäisches Patentamt

European Patent Office

Office européen des brevets

(11) Publication number: **0 262 753**
**A1**

## (12) EUROPEAN PATENT APPLICATION

(21) Application number: **87305037.1**

(22) Date of filing: **08.06.87**

(51) Int. Cl.⁴: **A61M 37/00 , A61L 15/03**

(30) Priority: **30.09.86 US 913207**

(43) Date of publication of application:
**06.04.88 Bulletin 88/14**

(84) Designated Contracting States:
**BE DE ES FR GB IT LU NL SE**

(71) Applicant: **PACO RESEARCH CORPORATION**
**1705 Oak Street**
**Lakewood New Jersey 08701(US)**

(72) Inventor: **Kim, Benjamin K.**
**226 Middle Drive**
**Toms River New Jersey 08753(US)**

(74) Representative: **Oliver, Roy Edward et al**
**POLLAK MERCER & TENCH High Holborn**
**House 52-54 High Holborn**
**London WC1V 6RY(GB)**

(54) Scopolamine transdermal delivery system.

(57) The present invention is directed to a transdermal delivery system for scopolamine. The delivery system is composed of multiple layers (14, 16, 18) containing a varying concentration of scopolamine base. Each layer (14, 16, 18) includes a medically acceptable rubber (such as a butyl rubber), an active substance (such as scopolamine) and a vehicle for the active substance (such as light mineral oil). Beginning with the first layer (in contact with the skin), concentration of the active substance increases from 7 % or less up to 10-16 %. This construction allows the controlled release of the active substance over a sustained period of time.

## FIG. I

## SCOPOLAMINE TRANSDERMAL DELIVERY SYSTEM

### Scope of the Invention

The present invention is directed to a transdermal delivery system. Scopolamine is the preferred active substance administered by the system. This delivery system has a constant release rate over a period of time.

### Background of the Invention

Recently transdermal delivery systems have become popular. These systems allow a controlled amount of an active substance, such as scopolamine, to be continuously administered over a sustained period of time. One example of these systems is found in U.S. Patent No. 4,031,984.

In U.S. Patent No. 4,031,984, the release rate of scopolamine from the delivery system is controlled by the mass transfer of the active substance through a microporous membrane. The scopolamine is present in a reservoir layer and an adhesive layer. However, absorption of the scopolamine through the skin is controlled by stratum corneum.

Scopolamine is a belladona alkaloid and widely distributed in nature, especially in the Solanaceae plants. Scopolamine is a muscarinic cholinergic blocking agent and has been used to prevent the nausea and vomiting of motion sickness.

### Summary of the Invention

The present invention is related to transdermal delivery system. The transdermal system is preferably used to administer scopolamine for the prevention of nausea and vomiting caused by motion sickness. The system comprises a matrix which is sandwiched between a release layer and a backing layer. The matrix includes a first layer having about 50 to about 60% of a medically acceptable rubber, about 7% or less of an active substance, and about 35 to about 45% of a vehicle for the active substance and being about 0.05 to about 0.10 mm thick. The matrix includes a second layer having about 45 to about 55% of a medically acceptable rubber, about 8% or less of the active substance and about 40 to about 50% of a vehicle for the active substance and being about 0.02 to about 0.07 mm thick. The matrix includes a third layer having about 45 to about 55% of a medically acceptable rubber, about 10 to about 16% of the active substances and about 32 to about 39% of a vehicle for the active substance and being about 0.02 to about 0.09 mm thick.

### Description of the Drawings

For the purpose of illustrating the invention, there is shown in the drawings a form which is presently preferred; it being understood, however, that this invention is not limited to the precise arrangements and instrumentalities shown.

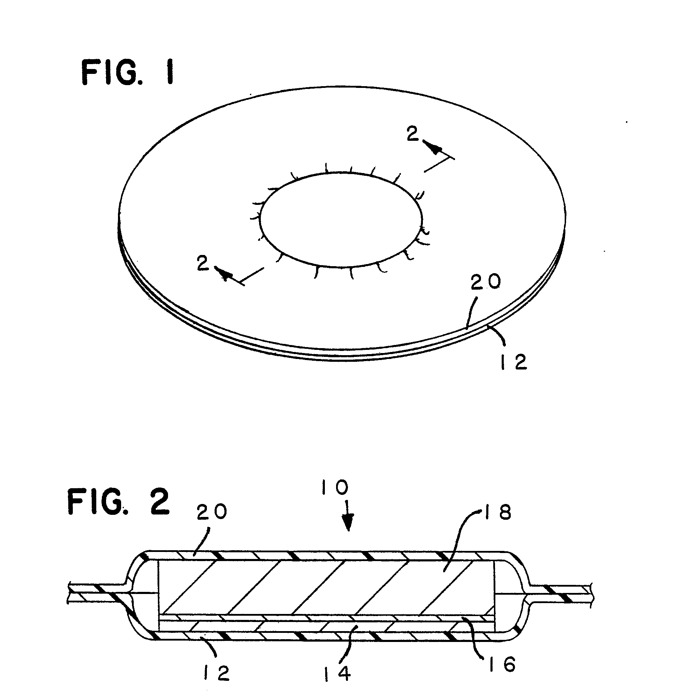

Figure 1 is an isometric view of a preferred embodiment of the present invention.

Figure 2 is a sectional view taken generally along lines 2-2 in Figure 1.

### Detailed Description of the Invention

The transdermal delivery system comprises a polymeric matrix which is preferably formed by three thin and discrete layers. Each layer contains a different concentration of an active substance. The release rate of the active substance is controlled by the different concentration in each layer, a variable amount of vehicle, such as light mineral oil, and the different thickness of each layer.

The matrix is composed on a weight percentage basis of the entire delivery system from about 45% to 65% of a medically acceptable rubber (preferably a butyl rubber such as isobutylene-isoprene copolymers), from about 4 to 15% of an active substance (preferably scopolamine) and from about 30 to 50% of a vehicle for the active substance (preferably a light mineral oil USP).

Referring to the drawings, wherein like numerals indicate like elements, there is shown a transdermal delivery system made according to the present invention and denoted 10.

A layer 12 is the lowermost layer of system 10. Layer 12 is preferably made of a silicon coated polyethylene release material. Layer 12 is impermeable to the scopolamine base and other components of the matrix. Additionally, layer 12 fulfills a support function and a protective function. Layer 12 is removed from the system 10 prior to applying the system to the skin.

Layer 14, or the first layer of the matrix, is for direct face to face contact with the skin. Layer 14 is composed of about 50 to 60% of a medically acceptable rubber, about 7% or less of the active substance and about 35 to 45% of a vehicle for the active substance. Layer 14 is preferably between about 0.05 mm and 0.10 mm thick.

Layer 16, or the second layer of the matrix, is composed of about 45 to 55% of a medically acceptable rubber, about 8% or less of the active substance and about 40 to 50% of the vehicle for the active substance. Layer 16 is preferably between about 0.02 mm and 0.07 mm thick.

Layer 18, or the third layer of the matrix, is composed of about 45% to 55% of a medically acceptable rubber, about 10 to 16% of the active substance and about 32 to 39% of the vehicle for the active substance. Layer 18 is preferably between about 0.02 mm and 0.09 mm.

Layer 20, or the uppermost layer of the delivery system, is a protective backing, preferably composed of an aluminized polyethylene film. Layer 20 prevents the active substance and other substances from escaping from the system.

Layers 14, 16 and 18 control the active substance release rate to obtain the optimum therapeutic blood level of the active substance. The release rate is a function of the active substance diffusion through each layer, the thickness of each layer, the concentration gradient of active substance in the layers and the diffusion coefficient of the active substance in the vehicle. The concentration gradient depends on the active substance concentration in the vehicle in the next layer.

Scopolamine is the preferred active substance. Scopolamine is a highly potent anticholinergic agent and optimum drug release rate is a critical factor to prevent the nausea and vomiting of motion sickness and to eliminate the side effects, such as tachycardia, drowsiness and dry mouth. Those skilled in the art will recognize that other active substances could be used in the present invention.

A preferred medically acceptable rubber is isobutylene-isoprene copolymer (Exxon 077) with molecular weight more than 300,000, manufactured by Exxon Chemical Company, a division of Exxon Corporation, Houston, Texas. This butyl rubber (Exxon 077) contains no more than 3.0 molecular percent isoprene and has been used as chewing gum base. It is a hydrophobic polymer and allows slow diffusion of the active substance. The vehicle (oil)-butyl rubber mixture is an excellent adhesive and helps to bind the multiple layer matrix. Alternately, polyisobutene (Exxon Vistanex MM) or ethylene-propylene copolymer (Exxon Vistalon) can be used as the medically acceptable rubber. Those skilled in the art will recognize that other medically acceptable rubbers could be used in the present invention.

The vehicle of this transdermal delivery system is preferably light mineral oil which is listed in the U.S. Pharmacopeia XXI. Alternately, the combination of silicon oil, vegetable oil, isopropyl palmitate and isopropyl myristate with mineral oil can be used as a vehicle in this system. Those skilled in the art will recognize that other vehicles could be used in the present invention.

This delivery system may be applied behind the ear (mastoidal region) and it will administer scopolamine according to the describe dosage amount.

### EXAMPLE 1

To form the third layer, 10g of butyl rubber (Exxon 077) is dissolved in 60ml of toluene with an agitator. 3.15g of scopolamine base and 7g of light mineral oil USP are added to the rubber/toluene mixture and mixed for 30 minutes to obtain a uniform solution. This solution is casted onto an approximately 0.07mm thick backing film of aluminized polyethylene (3M-Scotchpak 1006) to produce a third layer approximately 0.035mm thick.

To form the second layer, 11g of butyl rubber (Exxon 077) is dissolved in 65ml of toluene with a proper agitator. 0.245g of scopolamine base and 6g of light mineral oil USP are added to the rubber/toleune mixture and mixed for 30 minutes to obtain a uniform solution. This solution is casted onto a silicon coated polyethylene film (3M-1022) or silicon coated paper (H.P. Smith bleached poly silk #8024. Division of James River Corporation, Bedford Park, Illinois) to produce a second layer approximately 0.035mm thick.

To prepare the first layer of the patch, 9g of butyl rubber (Exxon 077) is dissolved in 60ml of toluene with a proper agitator. 0.75g of scopolamine base and 7g of light mineral oil are added to the polymer solution and mixed for 30 minutes. This solution is casted onto a silicon coated polyethylene film (3M-1022) or silicon coated paper (H.P. Smith bleached poly silk #8024) approximately 0.07mm thick.

The second layer and first layer are overlapped on the third layer. The top of this matrix is covered with silicon coated polyethylene film and 2.5 cm² circular, disc shaped patches are punch cut. This patch is designed to release scopolamine continuously 2.5-3.0 ug/cm²/hr. for 72 hours.

## EXAMPLE 2

To form the third layer, 50g of butyl rubber (Exxon 077) solution in chloroform, 6g of light mineral oil and 1.35g of scopolamine base are mixed for 30 minutes with a proper agitator. This solution is coated onto an approximately 0.07mm thick backing film of aluminized polyethylene (3M-Scotchpak 1006) to produce a third layer approximately 0.035mm thick.

To form the second layer 50g of butyl rubber (Exxon 077) solution in chloroform, 6g of light mineral oil USP and 0.225g of scopolamine base are well mixed for 30 minutes with a proper agitation. This solution is coated onto a silicon coated polyethylene film (3M-1022) or silicon coated paper (H.P. Smith bleached poly silk #8024) to produce a second layer approximately 0.035mm thick.

To form the first layer, 50g of butyl rubber (Exxon 077) solution in chloroform, 7.5g of light mineral oil USP and 0.25g of scopolamine base are mixed well for 30 minutes with a proper agitator. The casting procedure is followed as above procedure.

The scopolamine patch is prepared by the same method as described in Example 1.

## EXAMPLE 3

The third layer of the scopolamine patch is composed with 250mg of scopolamine base, 10g of butyl rubber (Exxon 077) solution and 1g of light mineral oil USP. The next steps are the same as Example 1.

The second layer of this patch is composed of 8.2mg of scopolamine base, 10g of butyl rubber (Exxon 077) solution and 1g of light mineral oil USP. The next steps are the same as Example 1.

The first layer of the patch is composed of 10g of butyl rubber (Exxon 077) solution and 1.2g of light mineral oil. Scopolamine base is not contained in this layer. The next steps are the same as above.

These three layers are overlapped and the patch is prepared following the Example 1 procedure.

The present invention may be embodied in other specific forms without departing from the spirit or essential attributes thereof and, accordingly, reference should be made to the appended claims, rather than to the foregoing specification, as indicating the scope of the invention.

## Claims

1. A transdermal delivery system characterized by:

(a) a matrix having

(i) a first layer (14) including about 50 to about 60% of a medically acceptable rubber, about 7% or less of an active substance, and about 35 to about 45% of a vehicle for said active substance, said first layer having a thickness of about 0.05 to about 0.10 mm;

(ii) a second layer (16) including about 45 to about 55% of a medically acceptable rubber, about 8% or less of said active substance, and about 40 to about 50% of a vehicle for said active substance, said second layer having a thickness of about 0.02 to about 0.07 mm;

(iii) a third layer (18) including about 45 to about 55% of a medically acceptable rubber, about 10 to about 16% of said active substance and about 32 to about 39% of a vehicle for said active substance, said third layer having a thickness of about 0.02 to about 0.09 mm;

(b) a release layer (12) disposed on a face of said first layer (14); and

(c) a backing layer (20) disposed on a face of said third layer (18), whereby said release layer (12) and said backing layer (20) sandwich said matrix.

2. The system according to claim 1 wherein said active substance is scopolamine base.

3. The system according to claim 1 wherein said medically acceptable rubber is selected from the group consisting of isobutylene-isoprene copolymer, polyisobutene, or ethylene-propylene copolymer.

4. The system according to claim 1 wherein said vehicle is selected from the group consisting of light mineral oil or a mixture of silicon oil, vegetable oil, isopropyl palmitate and isopropyl myristate with mineral oil.

5. The system according to claim 1 wherein said release layer is a silicon coated relese material.

6. The system according to claim 1 wherein said backing layer is an aluminized material.

7. The system according to claim 1 wherein said face of said matrix has a surface area of about 1.0 to about 5.0 cm$^2$.

8. A matrix for a transdermal delivery system characterized by:

(a) a first layer (14) including about 50 to about 60% of a medically acceptable rubber, about 7% or less of an active substance, and about 35 to about 45% of a vehicle for said active substance, said first layer having a thickness of about 0.05 to about 0.10 mm;

(b) a second layer (16) including about 45 to about 55% of a medically acceptable rubber, about 8% or less of said active substance, and about 40 to about 50% of a vehicle for said active substance, said second layer having a thickness of about 0.02 to about 0.07 mm;

(c) a third layer (18) including about 45 to about 55% of a medically acceptable rubber, about 10 to about 16% of said active substance and about 32 to about 39% of a vehicle for said active substance, said third layer having a thickness of about 0.02 to about 0.09 mm.

9. The matrix according to claim 8 wherein said active substance is scopolamine base.

10. The system according to claim 1 wherein said medically acceptable rubber is selected from the group consisting of isobutylene-isoprene copolymer, polyisobutene, or ethylene-propylene copolymer.

11. The system according to claim 1 wherein said vehicle is selected from the group consisting of light mineral oil or a mixture of silicon oil, vegetable oil, isopropyl palmitate and isopropyl myristate with mineral oil.

12. A transdermal delivery system characterized by:

(a) a matrix having

(i) a first layer (14) including about 50 to about 60% of a medically acceptable rubber, about 7% or less of scopolamine base, and about 35 to about 45% of a vehicle for said scopolamine base, said first layer having a thickness of about 0.05 to about 0.10 mm;

(ii) a second layer (16) including about 45 to about 55% of a medically acceptable rubber, about 8% or less of said scopolamine base, and about 40 to about 50% of a vehicle for said scopolamine base, said second layer having a thickness of about 0.02 to about 0.07 mm;

(iii) a third layer (18) including about 45 to about 55% of a medically acceptable rubber, about 10 to about 16% of said scopolamine base, and about 32 to about 39% of a vehicle for said scopolamine base, said third layer having a thickness of bout 0.02 to about 0.09 mm;

(b) a release layer (12) disposed on a face of said first layer (14); and

(c) a backing layer (20) disposed on a face of said third layer (18), whereby said release layer (12) and said backing layer (20) sandwich said matrix.

13. The system according to claim 12 wherein said medically acceptable rubber is selected from the group consisting of isobutylene-isoprene copolymer, polyisobutene, or ethylene-propylene copolymer.

14. The system according to claim 12 wherein said vehicle is selected from the group consisting of light mineral oil or a mixture of silicon oil, vegetable oil, isopropyl palmitate and isopropyl myristate with mineral oil.

## FIG. 1

## FIG. 2

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| A | EP-A-0 144 486  (HOFFMANN et al.)<br>* Abstract; figures 1,2; page 6, paragraph 2; page 7, paragraphs 2-4; page 10, lines 1-10 *<br>--- | 1,8,12 | A 61 M  37/00<br>A 61 L  15/03 |
| A,D | US-A-4 031 894  (URQUHART et al.)<br>* Whole document *<br>--- | 12 | |
| A | EP-A-0 178 140  (CHENG et al.)<br>* Figure 2; page 6, lines 2-12,17-27; page 7, lines 1-3 *<br>----- | | |

| | |
|---|---|
| | TECHNICAL FIELDS SEARCHED (Int. Cl.4) |
| | A 61 L<br>A 61 M |

The present search report has been drawn up for all claims

| Place of search | Date of completion of the search | Examiner |
|---|---|---|
| THE HAGUE | 18-12-1987 | FISCHER G.H. |